# EUROPEAN PATENT APPLICATION

(11) **EP 4 292 622 A1**
(43) Date of publication of application: **20.12.2023**
(21) Application number: 22179345.8
(22) Date of filing: 16.06.2022
(51) Int. Cl.: A61M 1/06, A61B 5/00

(54) **A SYSTEM FOR IDENTIFYING A BREAST, FOR A DEVICE WHICH APPLIES A FUNCTION TO A BREAST**

(71) Applicant: Koninklijke Philips N.V., 5656 AG Eindhoven (NL)
(72) Inventor: CLAASSEN, Coen Petrus Martinus, Eindhoven (NL); JOOSTEN, Franciscus Ivo, Eindhoven (NL); BENTVELSEN, Petrus Henricus Cornelius, Eindhoven (NL); DOBRUSSKIN, Christoph, Eindhoven (NL); VAN VELDHUIZEN, Gijsbert Hendrik, Eindhoven (NL)
(74) Representative: Philips Intellectual Property & Standards

(57) **Abstract**

A device is for mounting over a breast to apply a function to the breast. The device has a sensor arrangement for determining, independently of any device mounted on the other breast, if the device is mounted over the left or right breast of a user.

## Description

### FIELD OF THE INVENTION

This invention relates to devices to be mounted over a breast for applying a function, such as a milk expression function and/or a massage function.

### BACKGROUND OF THE INVENTION

Breast pumps are used by breast feeding women to extract milk from their breast such that the extracted milk can be fed to their babies at a later time. It is also known for breast pumps to incorporate a breast massage function. Devices only for performing a massage function are also known.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup, called a breast shield, and a vacuum is applied such that milk is extracted. A motorized breast pump typically has two operating modes, namely a stimulation mode and an expression mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the expression mode, which has a lower frequency (typically around 1Hz) and higher intensity pumping (i.e. a deeper vacuum with more under pressure) for more effective milk expression.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps. Recently, there is a trend towards wearable breast pumps. These devices generally have a smaller form factor and can be fully worn on the user's body, allowing the user to move around freely. Some of these are small enough to fit into a user's bra. A motor, battery and milk collection container are for example integrated to form a single unit.

Expressing mothers may want to log pumping data, and/or to have the breast pumps adapt settings automatically, such as vacuum levels or cycle times, to provide a personalized and optimized performance.

It has been recognized that it would be useful to log breast pump related data, for example the amount of milk expressed, the time at which the milk was expressed etc. For example, US 2021/205511 discloses a breast pump which communicates pump related data to an external device, to separately track and record pumping and milk expression data for the left and right breasts.

It would also be of interest to be able to apply settings depending on the breast, for example breast pumping vacuum levels. This automated collection of data and/or adaptation of settings would also be of interest for other functions, such as a breast massage function. For example, the data relating to the times and durations may be collected, or massage settings may be configured for each breast.

Currently, for a system with a single device which can be applied to either breast, the user is required to identify the breast to which the device has been applied.

### SUMMARY OF THE INVENTION

The invention is defined by the claims.

According to examples in accordance with an aspect of the invention, there is provided a system for identifying a breast, comprising:
a device for mounting over a breast to apply a function to the breast, comprising a sensor arrangement; and
a processor for interpreting an output signal generated by the sensor arrangement, thereby to determine independently of any device mounted on the other breast, if the device is mounted over the left or right breast of a user.

The sensor arrangement and processor thus together determine if the device is mounted over the left or right breast of a user. The sensor arrangement is able to do so independently of any device mounted on the other breast.

This device is able to detect automatically the breast (left or right) of a particular user to which it has been mounted.

In a first set of examples, the device can perform this detection if it is the only device in use.

The device is for example trained in a calibration phase such that the sensor arrangement can recognize or otherwise determine which breast it has been mounted to. This enables the device performance to be monitored for the particular breast, and/or device settings to be applied automatically for the particular breast.

The independent sensing means that no information is needed to be communicated from a device over the other breast, in particular no information is needed which indicates whether the another device is mounted over the left or right breast.

The sensor arrangement is for example for sensing characteristics of the breast.

The sensor arrangement is for example for sensing shape, size, surface, color or reflection characteristics of the breast or nipple. Thus, visual or other physical differences between the breasts can be detected.

The sensor arrangement may instead be for sensing a heartbeat. The two breasts have different distances from the heart the so that device can recognize on which breast the device has been mounted. A calibration phase may be used to get better results

The sensor arrangement may be for sensing a tilt angle or movement of the device. The device may not sit vertically in use, but may have a tilt angle which depends on the breast.

The sensor arrangement may also have the capability of determining, when there is another device on the other breast, on which side of that other device the device is located. Thus, sensing enables the device to detect another device on the other side and derive from that sensing the side on which it is mounted. This is an additional sensing capability to the main, independent, sensing capability.

The sensor arrangement may be for determining, when there is another device mounted on the body of the user in a known body position, a location of that other device relative to the device.

In this way, the sensor arrangement may determine if the device is mounted over the left or right breast using a reference associated with a position on a user's body other than a position on the other breast. For instance, the device may determine if it is mounted over the left or right breast using a further device worn by the user in a known position (such as around the waist or on a particular wrist).

If no other device is sensed, the default operation is to determine the left/right side in isolation.

The left/right detection can be continuous or intermittent.

In one set of examples, the device comprises a breast pump, wherein the function comprises milk expression. The device may thus collect milk expression information, which can then be saved as data associated with one particular breast. Based on the detected side, breast pump operating parameters may be automatically set to match user's preferences.

The sensor arrangement is for example for sensing a nipple elongation characteristic during breast pumping. The two nipples may have different elongation characteristics, and these differences can be detected.

The sensor arrangement may also be for sensing a milk expression characteristic during breast pumping. A memory may then be provided for storing milk expression performance for the breast on which the device is mounted, or else a communications system may be provided for communicating data to be stored in remote memory.

The expression performance data can be used to make suggestions to improve the comfort or performance of the breast milking experience or to modify pumping parameters or times. In general the collected data can be used to give advice to a user.

In another set of examples, the device comprises a breast massage device, wherein the function comprises breast massaging.

In another set of examples, the device comprises a light treatment device, wherein the function comprises delivery of light.

In another set of examples, the device comprises a topical application device, wherein the function comprises application of a topical.

The device may then have a blood perfusion sensor. This may be used to monitor the results of the massage function.

In all examples, the device may comprise a communications system for communicating which breast has been identified to a remote device. Thus, a remote device, such as a mobile phone, may be used to log data relating to the use of the device.

For this purpose, the sensor arrangement may be further for sensing performance characteristics of the function applied by the device, and the communications system is for communicating the performance characteristics to the remote device. The performance characteristics may be milk expression settings or performance or massage device settings or performance.

The invention also provides a system for providing a function to a breast, comprising:
the device as defined above; and
software for loading on a remote device to enable the remote device to receive the determined breast,
wherein the software enables the remote device to communicate with another device mounted on the other breast.

In this way, one device can communicate with another, via the remote device (e.g., mobile phone).

The invention also provides a computer program comprising computer program code which is adapted, when said program is run on a computer, causes the computer to implement a method comprising:
receiving sensor data from a sensor arrangement of a device for mounting over a breast to apply a function to the breast; and
determining from the sensor data if the device is mounted over the left or right breast of a user independently of any device mounted on the other breast.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows the invention as applied to a wearable breast pump;
Figure 4 shows possible differences between heartbeat readings;
Figure 5 shows how a breast pump may not sit vertically in use, but may have a tilt angle which depends on the breast;
Figure 6 shows that a device can determine that it is the left or right device if there is another device to opposite side;
Figure 7 shows a sensor arrangement for left/right detection and a separate sensor arrangement for sensing milk expression characteristics during breast pumping; and
Figure 8 shows the system of Figure 7 with the memory as part of a remote device such as a mobile phone.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for mounting over a breast to apply a function to the breast. The device has a sensor arrangement for determining, independently of any device mounted on the other breast, if the device is mounted over the left or right breast of a user. The device may be a breast pump or a breast massage device, or a device for applying any other function, e.g. a breast tissue analysis device.

Examples of possible devices include:
(i) breast shields that measure the amount of milk fed to a baby.
(ii) devices for general breast or nipple light, thermal or topical treatment.
(iii) devices for inverted nipple treatment.
(iv) a milk collection device.

A treatment device such as a breast massage device may for example massage a specific location for each breast based on the identification of the breast at which the device is situation (e.g. medial or lateral sides).

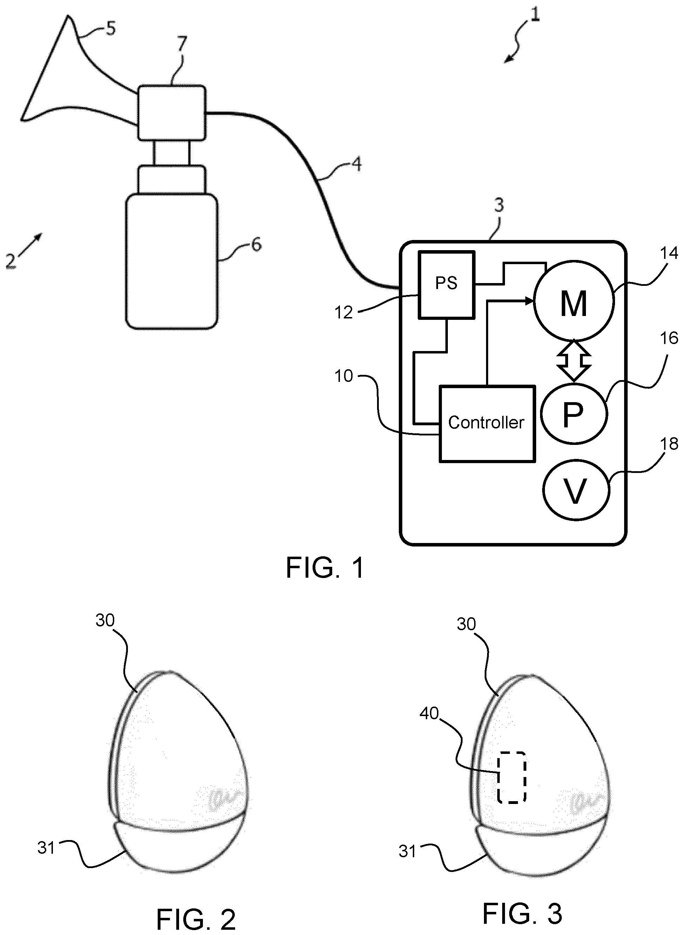

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the diaphragm prevents expressed milk from flowing directly into the pump arrangement 3.

The pump arrangement 3 comprises a controller 10, a power source 12, a motor 14 and a vacuum pump 16 (i.e. the impeller driven by the motor 14). A membrane pump (displacement pump) may instead be used. The controller controls 10 the operation of the power source 12, motor 14 and vacuum pump 16. The pump arrangement 3 further comprises a solenoid valve 18.

In use, the vacuum pump 16 applies a vacuum to the diaphragm located in the main body 7 so that it deforms. The diaphragm deforms to create a vacuum in the funnel 5, when it is sealed to the breast, which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of a known operation of a standard breast pump system. Other breast pump systems are of course known.

Breast pumps may also include a massage function, for example using a motor to create a vibration (e.g. WO 2020/178737). This can be soothing, either during or before or after milk expression. It can also be used to promote milk expression. Breast pumps may also include a light treatment function.

There are also breast pumps that have expression cups to be worn under a bra. There are also fully wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

There are also other types of wearable breast pump whereby the expression unit is fixed to a breast and the pump unit and/or milk collection container are situated elsewhere on the body of a user.

Figure 2 shows a wearable breast pump, comprising an expression kit 30 attached to a respective milk collection container 31. The breast pump may for example fit into a bra.

The invention can be applied to any existing type of breast pump with or without a massage function, or to a breast treatment device such as a massaging device, a light treatment device, a heat treatment device or a device for application of topicals such as cream. Thus, generally the invention relates to any device which is to be mounted on the breast to apply a function to the breast. The device of the invention has a sensor arrangement for determining, independently of any device mounted on the other breast, if the device is mounted over the left or right breast of a user.

Figure 3 shows the invention as applied to a wearable breast pump. The invention involves the addition of a sensor arrangement 40 for determining if the breast pump is mounted over the left or right breast of a user. The sensor arrangement includes a sensor element, and there are various examples of possible sensor element described below. A processor processes the sensor output data to make the determination. The processor may be part of the sensor arrangement itself so that they form a single unit which is part of the breast-mounted device. However, the processing of the sensor data may be performed remotely by a remote device with which the sensor arrangement communicates.

The determination can be made in isolation, in others words irrespectively of whether or not another device is attached to the other breast. Thus, the sensor arrangement and the associated processor can determine from characteristics of, or associated with, the breast on which it is mounted whether it is the left or right breast of the user. In some, but not all, cases, this may involve an initial learning phase by which the sensor arrangement learns the difference between the left and right breasts of the particular user in respect of the characteristics that are detected by the sensor arrangement.

There various options for the sensor arrangement 40, and these options may be used alone or in combination to create a more robust detection. The sensor arrangement 40 is only shown schematically in Figure 3 since it may take many different forms as discussed below.

In the case of a breast pump, user performance (e.g. milk expression statistics) can be collected for the particular breast, or device settings or performance for that particular breast, such as a maximum vacuum level or time durations, may be automatically chosen.

In a first set of examples, the sensor arrangement is for example for sensing physical or physiological characteristics of the breast. For this purpose, the sensor arrangement may comprise an image sensor for capturing visual images or the breast or nipple, or force sensors for capturing contact forces at different locations which may characterize the shape of the breast. These sensors may capture shape, size, surface, and/or color and reflection characteristics of the breast or nipple. This information is then analyzed by the processor.

Dynamic movement observed on the left and right breast may also not be identical and so enable to a left/right determination to be made. Acceleration sensors may be used for this purpose, and the acceleration over time is translated into velocity and distance covered by the device. The acceleration sensor for example comprises an accelerometer and/or gyroscope.

In a second set of examples, the sensor arrangement comprises an accelerometer for detecting motion caused by the heartbeat of the user or a microphone for detecting heartbeat sounds.

For motion signals, with suitable band pass filtering to the possible range of frequencies of the heartbeat by the processor, a heartbeat signal can be extracted.

The two breasts have different distances from the heart, so that with a calibration phase as mentioned above, the device can recognize on which breast the device has been mounted based on e.g. the amplitude of the heartbeat signal. The reference information obtained during the calibration phase is stored in a database. The calibration phase is for example the first use of the device, whereby the user assigns a left and right label to the device used on the respective side.

A learning algorithm may be used that, after a few uses and/or user input, recognizes which side the device is on, on its own.

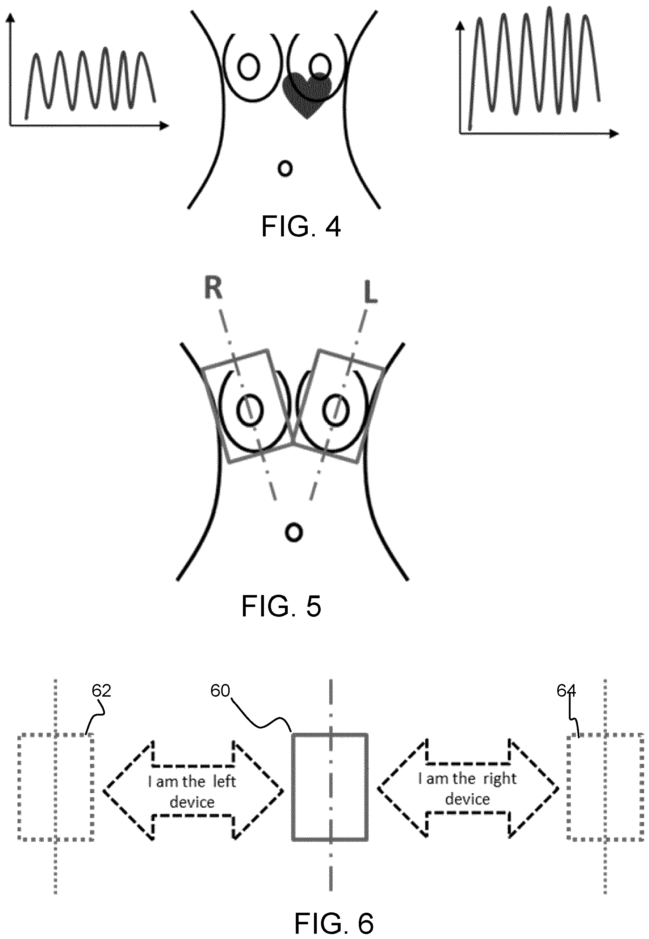

Figure 4 shows possible differences between heartbeat readings of the sensor arrangement on the left and right side of the user. Such differences may be detected as amplitude differences, time-shifts, profiles or a combination thereof.

In a third set of examples, the sensor arrangement may comprise a tilt sensor for sensing a tilt angle of the device (relative to the gravity vector). This may also use an accelerometer, or an inertia monitoring unit (IMU), for example with an accelerometer and gyroscope and optionally also a magnetometer. The IMU thus combines multiple inertial measurement devices.

Figure 5 shows how the breast pump may not sit vertically in use, but may have a tilt angle which depends on the breast. This tilt angle may be left/right or front/back or a combination.

In a fourth set of examples, the sensor arrangement is for sensing nipple elongation characteristics during breast pumping. The vacuum profile delivered during operation of breast pump results in manipulation of the breast and in particular creates nipple stretching. The two nipples may have different elongation characteristics, and these differences can be detected. The sensor arrangement for this purpose for example comprises an optical sensor for detecting the maximum forward extent of the nipple, indicating the maximum nipple elongation. The same sensor may detect a nipple size at rest.

The nipple stretching characteristics are again collected during a calibration phase, and differences are logged for each of the left and right breasts. During the subsequent sessions the nipple stretching of the breast on which the device is placed is measured, and the result is compared by the processor with the data from the previous sessions.

As explained above, the sensor arrangement in one set of examples can detect the breast on which it is mounted without reference to any other device.

In another set of examples, the sensor arrangement has additional functionality which is used when when there is another device on the other breast. It can for example detect the presence of another device on the other breast, and then by determining on which side of that other device the device is located, it is possible to determine which breast is associated with which device. Thus, the device may "look" to the other side to detect if there is another device on the other side. The positional relationship between two devices enables one device to derive from the other the side on which it is mounted. This may improve the detection reliability and ease when there are two devices mounted.

Thus, a single device may have both functions, namely the sensor arrangement and associated processor may be able to detect the left/right location in isolation as well as being able to derive information from the presence of another device on the other breast.

Figure 6 shows that a device 60 can determine that it is the left device if there is another device 62 to the right (looking forwards, whereas Figure 6 shows a view looking towards the user). Similarly, the device 60 can determine that it is the right device if there is another device 64 to the left.

Only one device of a pair then needs to be enabled with the function of detecting the side on which it is mounted in isolation. The other device may then only need to detect the side with reference to the other already-mounted device.

The detection of the other device may make use of an RFID tag/chip. For example the device may have an RFID interrogation system, and it can then detect the presence of a device on the other breast by interrogating the RFID tag/chip of that other device, and recognizing form which side of the device the RFID response was received. This detection can be performed without any active involvement of the other device. In other words, the other device does not sent any communication. Instead, the device detects the presence of the other device passively.

The sensor arrangement may determine if the device is mounted over the left or right breast using a reference associated with a position on a user's body other than a position on the other breast. For instance, the device may determine if it is mounted over the left or right breast using a further device worn by the user is wearing. Such a further device may be a pump for expressing milk. The belt and/or hardware connections between the device and the further device may be arranged such that the further device must be worn in a specific location on the user's body, thereby creating a reference. The further device may be a reference mounted on a bra the user is wearing, like an RFID tag.

Instead of an RFID tag, the sound of the other breast pump may be captured by a microphone.

The further device may be a smart watch which is worn at a known position (because the user tells it which wrist it is worn or because it detects which wrist it is on).

The two devices may thus be identical or they may be different. The detection can be implemented using a sensor arrangement in the form of a (directional) microphone, a Hall sensor, sensor or a Bluetooth low energy antenna.

These examples are based on distinguishing left from right based on asymmetries detected by sensor data. These asymmetries may be related to the user's physiology such as the shape or size of a nipple, or may be related to other user related characteristics such as the heartbeat. However, asymmetries may also be related to user-external factors such as the left device sensing the location of the right device or indeed of any other device also worn on the body.

The sensing arrangement may alternatively or additionally be based on historical data collected over time. For example, for the case of a breast pump, milk production differences over time (such as milk flow, or onset of the milk ejection reflex, MER) can be logged by the system.

For this purpose, the sensor arrangement may comprise a flow sensor. This may be an optical sensor for sensing milk flow, or a weight sensor for sensing a collected milk weight, or a level sensor such as a capacitive level sensor, or any other suitable known sensor able to monitor the milk collection.

The vacuum versus time characteristics may be monitored based on the voltage or current of the vacuum pump motor over time. For example, when the current exceeds a threshold this relates to the expression time.

The vacuum pump power consumption will change based on the load that is placed on the pump. The load changes when the pumped volume is changed. For example, when there is an air leak the volume increases, when the nipple expands the volume decreases, or when milk enters the membrane and partially remains in the membrane the volume decreases.

Power consumption can be monitored using measurements of current and/or voltage. If the voltage is known (e.g. controlled by software) and the current can be measured, for example by means of a voltage divider, the current measurement can be used to determine the power consumption.

If there are consistent differences between the left and right breasts, or a returning pattern occurs, this can be used to distinguish between left and right.

The system can learn differences like this from a calibration phase as explained above, for example using additional user input to assign the respective data to the left or right breast. Knowing that the left breast has already been emptied recently would indicate the current breast being emptied is the right breast. The breast pump in this way collects milk expression information, which can then be saved as data associated with one particular breast.

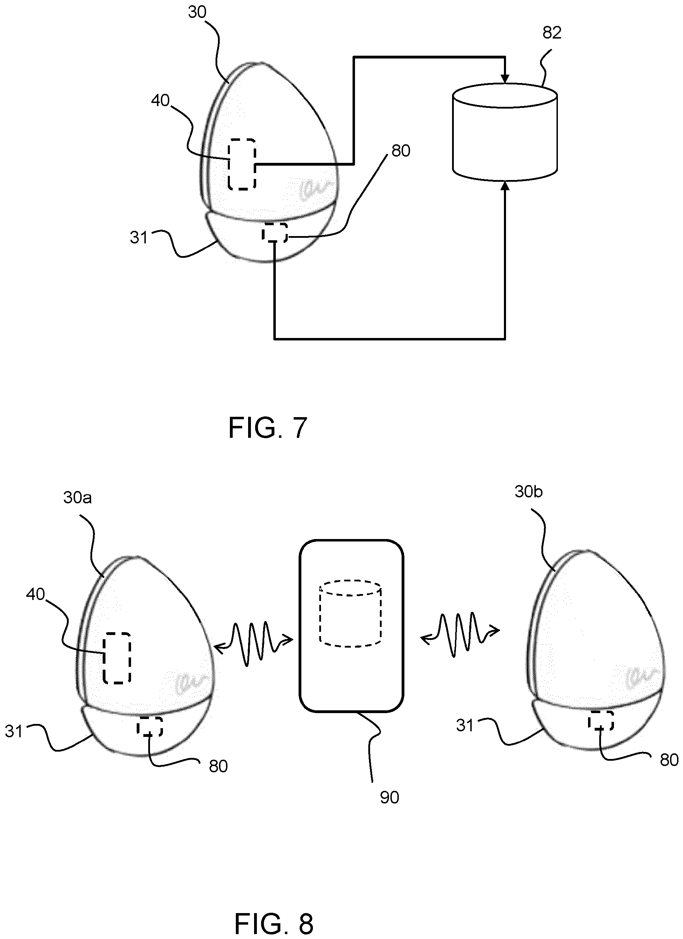

Figure 7 shows the sensor arrangement 40 for left/right detection (i.e. any of the options above) and shows a separate sensor arrangement 80 for sensing milk expression characteristics during breast pumping. A memory 82 may then be provided for storing milk expression performance for the breast on which the device is mounted. The memory can also store user preferences, such as preferred breast pump settings (e.g. maximum vacuum level) for each breast, or preferred settings for other types of device such as massage force settings.

This memory may be part of the breast pump or it may be a remote device with which the breast pump communicates, e.g. using Bluetooth or WiFi. The expression performance data can be used to make suggestions to improve the comfort or performance of the breast milking experience or to modify pumping parameters or times. In general the collected data can be used to give advice to a user.

Another aspect of the invention relates to the communication of data relating to one breast-mounted device to a remote device or indeed to another breast-mounted device. This communication should be implemented in a cost-effective way, and it may be used to modify a control parameter for a device (such as a pumping or massaging parameter).

Figure 8 shows the system of Figure 7 with the memory 82 as part of a remote device 90 such as a mobile phone. A first device 30a detects the breast on which it is mounted, e.g. the left breast. This information is then transmitted to the remote device 90. The remote device 90 for example stores this information for later use, for example to add use-related data such as duration of use, or the amount of milk expressed, linked to the use of the device on the left breast.

The remote device may also receive use-related data from a second device 30b used by the user, and automatically assign those data to the other breast (hence not needing the second device 30b to include left-right detection).

It may further inform the second device 30b which breast it is on. Thus, in this example, only one of the two devices needs to have a sensor or system to determine its position on the user.

The remote unit 90 may notify each device of personal preferences (or a personal profile which includes such preferences) such as the strength of the vacuum of a breast pump, or the strength of massage of a massaging unit, or a milk target level, or a massage location and/or duration. Thus, it may function to transfer user preferences from one device to the other. Other information may be stored, such as the date/time of session or the length of the session. An estimation of the remaining operating time based may also be made based on the usage. A user preference may for example comprise a position of the unit, vacuum level, etc.

The information collected by the remote device 90 may be displayed on the remote device to inform the user, or it may be first collated, processed upon, summarized, or similarly treated and the derived information be displayed to the user.

The remote device 90 which stores the personalized information may instead be a remote server, and a mobile phone may then simply function as a user interface.

The remote device 90 may also provide information or instructions to a device which is not a breast-mounted device. For example, a breast pump system may comprise two expression kits connected to a single pump unit. The remote device may then communicate with the single pump unit.

The remote unit 90 may be used to control a single breast pump (or a single massage unit) in different ways depending on the breast on which the device is mounted. Thus, different control information may be provided by the remote unit 90 at different times, when a single device is used initially on one breast and later on the other breast.

Instead of a remote unit, the information stored by remote unit as explained above may be stored within each device. Thus, the device then identifies which breast it has been mounted over and the devices stores both left and right preferences, last used settings etc. Once the breast has been identified, the device can switch to the preferred or last used settings for that breast.

Monitoring of various physiological parameters may also be integrated into the devices. For example, measurement of blood profusion may be used to indicate a degree of effectiveness of a massage. A physiological signal can be used to measure the milk ejection reflex so that a setting of a breast pump can be changed, such as initiating a change in mode, or adapting a vacuum intensity or vacuum profile. A measurement indicative of high or low stress levels can be used to give a recommendation to a user for example.

As mentioned above, various sensing techniques can be combined for more accurate sensing. The left/right information can be used to determine the relative performance of each breast and respective data can be collected and used to make suggestions to improve the comfort or performance of the breast milking experience or to modify pumping parameters or times. In general the collected data can be used to give advice to a user.

User feedback may be enabled, whereby the user creates annotated data that enables the system to learn and improve its prediction accuracy for the left or right breast. The remote device that receives the sensor data (both the left-right information and performance data) is for example a mobile phone. User input can be provided via linked devices or via buttons on the device.

In general, the functions explained above may be applied to breast pumps or massaging devices or breast pumps incorporating a massage function.

The invention involves the use of a "sensor arrangement" to determine on which breast the device is mounted. The sensor arrangement comprises at least a sensor element mounted on the breast, and a processor is used for interpreting the sensor element signals. This processor may be an integrated with the sensor arrangement (located at the device) but it may also be remote (e.g. hosted by a smartphone with which the sensor element communicates).

In the examples above, the sensor arrangement determines if the device is mounted over the left or right breast of a user independently of any device mounted on the other breast. The device may do this fully by itself or it may use information derived from another device being at a known location, not being the other breast. The other device may send data to the device after a trigger from the device or spontaneously.

It is also explained that the sensor arrangement may have an additional function of triggering a response from a device mounted on the other breast, if there is a device mounted on the other breast (for instance by interrogating an RFID chip in the latter device). Triggering a response means that the sensor arrangement does not merely receive sent information from another device, but that first some action must be taken in response to which information is derived.

In this alternative set of examples, the device does not rely on any unsolicited communication sent from that other device but it may sense the presence of that other device or solicit a communication.

When the device has both functions (independent and using the presence of another device on the other breast) the sensor arrangement can choose the sensing approach based on whether or not another device is detected on the other breast.

Another concept is to determine if the device is mounted over the left or right breast of a user based on the presence of a device on the other breast (as the main function rather than an additional function as explained above). This detection does not need to rely on any communication sent from that other device but it may be based on sensing the presence of that other device, using sound or by triggering a response from the other device e.g. by RFID tag interrogation.

According to this concept, there is provided a system for identifying a breast, comprising:
a device for mounting over a breast to apply a function to the breast, comprising a sensor arrangement; and
a processor for interpreting an output signal generated by the sensor arrangement, thereby to determine if the device is mounted over the left or right breast of a user bv:
   passively sensing a device mounted on the other breast; or
   triggering a response from a device mounted on the other breast.

Various possible sensor arrangements have been described. They may be used in any combination to improve reliability of the detection.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A system for identifying a breast, comprising:
a device (30) for mounting over a breast to apply a function to the breast, comprising a sensor arrangement; and
a processor for interpreting an output signal generated by the sensor arrangement, thereby to determine independently of any device mounted on the other breast, if the device is mounted over the left or right breast of a user.

2. The system of claim 1, wherein the sensor arrangement (40) is for sensing characteristics of the breast.

3. The system of claim 2, wherein the sensor arrangement (40) is for sensing one or more of shape, size, surface, color and reflection characteristics of the breast or nipple.

4. The system of any one of claims 1 to 3, wherein the sensor arrangement (40) is for sensing a heartbeat and/or sensing a device orientation or movement of the device.

5. The system of any one of claims 1 to 4, wherein the sensor arrangement (40) is further for determining:
when there is another device on the other breast, on which side of that other device the device is located; or
when there is another device mounted on the body of the user in a known body position, a location of that other device relative to the device.

6. The system of any one of claims 1 to 5, comprising a breast pump, wherein the function comprises milk expression.

7. The system of claim 6, wherein the sensor arrangement (40) is for sensing nipple elongation characteristics during breast pumping.

8. The system of claim 6 or 7, wherein the sensor arrangement (40) is for sensing a milk expression characteristic during breast pumping.

9. The system of any one of claims 6 to 8, comprising:
a memory (82) for storing milk expression performance for the breast on which the device is mounted; or
a communications system for communicating milk expression performance data for the breast on which the device is mounted to a remote device for storage in remote memory.

10. The system of any one of claims 1 to 6, having the function or additional function of at least one of:
a breast massage device, wherein the function comprises breast massaging; or
a light treatment device, wherein the function comprises delivery of light; or
a topical application device, wherein the function comprises application of a topical.

11. The system of any one of claims 1 to 10, further comprising a blood perfusion sensor.

12. The system of any one of claims 1 to 11, wherein the device comprises a communications system for communicating the output signal generated by the sensor arrangement, or the determined breast, to a remote device (90).

13. The system of claim 12, wherein the sensor arrangement is further for sensing a performance characteristic of the function applied by the device, and the communications system is for communicating the performance characteristics to the remote device.

14. A system for providing a function to a breast, comprising:
the system of any one of claims 12 to 13; and
software for loading on a remote device to enable the remote device to receive identification of the breast that has been determined by the system of any one of claims 12 to 13,
wherein the software enables the remote device to communicate with another device mounted on the other breast.

15. A computer program comprising computer program code which is adapted, when said program is run on a computer, causes the computer to implement a method comprising:
receiving sensor data from a sensor arrangement of a device (30) for mounting over a breast to apply a function to the breast; and
determining from the sensor data if the device is mounted over the left or right breast of a user independently of any device mounted on the other breast.
